# EUROPEAN PATENT APPLICATION

(11) **EP 3 554 107 A1**
(43) Date of publication of application: **16.10.2019**
(21) Application number: 17879412.9
(22) Date of filing: 06.03.2017
(51) Int. Cl.: H04W 4/90

(54) **EMERGENCY RESPONSE DEVICE**

(30) Priority: 07.12.2016 KR 20160165525
(71) Applicant: LG Electronics Inc., Seoul 07336 (KR)
(72) Inventor: CHO, Hyunjung, Seoul 06772 (KR); CHO, Seihyun, Seoul 06772 (KR); SHIN, Daekee, Seoul 06772 (KR); PARK, Sunjung, Seoul 06772 (KR); JEON, Chanhun, Seoul 06772 (KR); PARK, Hongmin, Seoul 06772 (KR); PARK, Taesu, Seoul 06772 (KR); LEE, Chulbae, Seoul 06772 (KR)
(74) Representative: Mooser, Sebastian Thomas
(86) International application number: PCT/KR2017/002386
(87) International publication number: WO 2018/105818

(57) **Abstract**

An emergency response device that can be worn on the neck of a user according to an embodiment of the present invention may comprise: a communication module for performing communication with an emergency management server or a mobile terminal; a sensing module for sensing a condition of a user wearing the emergency response device; and a processor for, when it is determined that the user is in a dangerous situation, on the basis of information on the sensed condition of the user, outputting information reporting the dangerous situation.

## Description

### Technical Field

The present disclosure relates to an emergency response device and more specifically, to an emergency response device that manages an user's health and well-being.

### Background Art

In modern society, discomforts in everyday human being life have been alleviated due to a steady development of technology in various fields. The development of technology has been done in many fields throughout the industry at the same time. Especially, the development of technology in the field of electronic devices is accelerating.

The electronic device has been light and miniaturized and is designed to consume low-power, and has improved circuit integration, execution speed, durability, etc. The electronic device may be multifunctional and have high-performance.

Especially, although the population becomes aged, a technology related to an emergency response field for an individual has not come up with the above development of technology in the other field. Thus, an efficient and prompt response has not been achieved.

In an event of a sudden incident or an accident, a prompt notification or an external assistance request is required. As for a technology related to the emergency response of the individual, a cell phone with an emergency notification hotkey or a terminal dedicated for reporting an emergency situation has been conventionally employed.

In the cell phone with an emergency notification hotkey, when the user pushes the hotkey, the user is connected to a predetermined person or the emergency event is reported to a police station or the like.

However, conventionally, in the event of the emergency, the user needs to press a certain button. Thus, there was a difficulty in automatically providing a service for emergency notification.

### Disclosure of Invention

### Technical Purpose

The present disclosure aims to provide an emergency response device that can automatically determine the user's emergency situation and manage the user's health and well-being.

The present disclosure is intended to automatically provide guidance for users' health.

### Technical Solution

In one aspect of the present disclosure, there is provided an emergency response device wearable on a user's neck, the device comprising: a communication module configured for communicating with an emergency management server or a mobile device; a sensing module configured for sensing state information of a user while the user is wearing the emergency response device; and a processor configured for: determining, based on the sensed state information of the user, whether the user is in an emergency event; and upon determination that the user is in an emergency event, outputting information indicating the emergency event.

In one embodiment, the processor is further configured for: upon determination that the user is not in an emergency event, outputting an alert signal to allow the user to be awakened; and upon non-reception of a reaction to the alert signal from the user within a predetermined time after outputting the alert signal, outputting a vibration indicating the emergency event.

In one embodiment, the device further comprises a vibration module, wherein the processor is further configured for outputting the alert signal using the vibration module.

In one embodiment, the processor is further configured for outputting the information indicating the emergency event to the emergency management server or the mobile device.

In one embodiment, the device includes: a middle neck portion having a curved cross sectional shape; a right bent portion bent downwardly from a right end of the middle neck portion; and a left bent portion bent downwardly from a left end of the middle neck portion.

In one embodiment, the device includes: an input and output module including a speakers and a microphone; and a battery module mounted on the middle neck portion for supplying power to the emergency response device.

In one embodiment, the input and output module is mounted on the right bent portion, wherein the battery module is mounted in a center region of the middle neck portion.

### Technical Effect

According to the various implementations of the present disclosure, when the user wears only the emergency response device alone, the user's emergency state may be easily grasped and rapidly reported.

Further, a risk situation of the user is more accurately determined, and thus, appropriate measures corresponding thereto can be performed quickly.

### BRIEF DESCRIPTION OF THE DRAWINGS

FIG. 1 shows a configuration of an emergency response system according to one implementation of the present disclosure.
FIG. 2 is a rear view of an emergency response device according to one implementation of the present disclosure.
FIG. 3 is a perspective view of an emergency response device according to one implementation of the present disclosure.
FIG. 4 shows a configuration of a mounting device for mounting an emergency response device thereon according to one implementation of the present disclosure.
FIG. 5 is an illustration of a state in which an emergency response device is mounted on a mounting device in accordance with one implementation of the present disclosure.
FIG. 6 is a sequence diagram to illustrate a method for operating an emergency response system according to one implementation of the present disclosure.

### DETAILED DESCRIPTIONS

Reference will now be made in detail to implementations of the present disclosure in conjunction with the attached drawings. The same reference numbers in different figures denote the same or similar elements, and as such perform similar functionality. Well-known methods, procedures, components, and circuits have not been described in detail so as not to unnecessarily obscure aspects of the present disclosure. Suffixes "module" and "unit" for components used in the following description are provided in consideration of each drafting of the specification and are used interchangeably with each other. The suffixes "module" and "unit" may not be intended to distinguish between the components having suffixes.

It will be understood that, although the terms "first", "second", "third", and so on may be used herein to describe various elements, components, regions, layers and/or sections, these elements, components, regions, layers and/or sections should not be limited by these terms. These terms are used to distinguish one element, component, region, layer or section from another element, component, region, layer or section. Thus, a first element, component, region, layer or section described below could be termed a second element, component, region, layer or section, without departing from the spirit and scope of the present disclosure.

In addition, it will also be understood that when a first element or layer is referred to as being present "on" a second element or layer, the first element may be disposed directly on the second element or may be disposed indirectly on the second element with a third element or layer being disposed between the first and second elements or layers. It will be understood that when an element or layer is referred to as being "connected to", or "coupled to" another element or layer, it can be directly on, connected to, or coupled to the other element or layer, or one or more intervening elements or layers may be present.

The terminology used herein is for the purpose of describing particular embodiments only and is not intended to be limiting of the present disclosure. As used herein, the singular forms "a" and "an" are intended to include the plural forms as well, unless the context clearly indicates otherwise. It will be further understood that the terms "comprises", "comprising", "includes", and "including" when used in this specification, specify the presence of the stated features, integers, operations, elements, and/or components, but do not preclude the presence or addition of one or more other features, integers, operations, elements, components, and/or portions thereof. As used herein, the term "and/or" includes any and all combinations of one or more of the associated listed items. Expression such as "at least one of" when preceding a list of elements may modify the entire list of elements and may not modify the individual elements of the list.

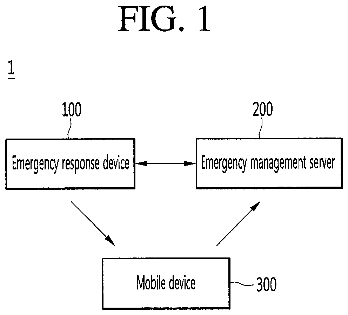

FIG. 1 illustrates a configuration of an emergency response system according to one implementation of the present disclosure.

Referring to FIG. 1, the emergency response system 1 may include an emergency response device 100, an emergency response server 200, and a mobile device 300.

The emergency response device 100 may be worn on the user's neck. The emergency response device 100 may be worn on the user's neck to sense the user's state.

The emergency response device 100 may generate state information of the user based on the detected state of the user and transmit the generated state information of the user to the emergency response server 200 or the mobile device 300.

The emergency response server 200 may receive the state information of the user from the emergency response device 100 or from a mounting device to be described later.

The mobile device 300 may receive the state information of the user from the emergency response device 100 or from a mounting device to be described later.

The mobile device 300 may be a device owned by a guardian for the user of the emergency response device 100. However, this is merely an example. The mobile device 300 may be a mobile device owned by the user of the emergency response device 100.

The emergency response system 1 may further include a mounting device for mounting the emergency response device 100 thereon.

Next, a structure of the emergency response device 100 will be described.

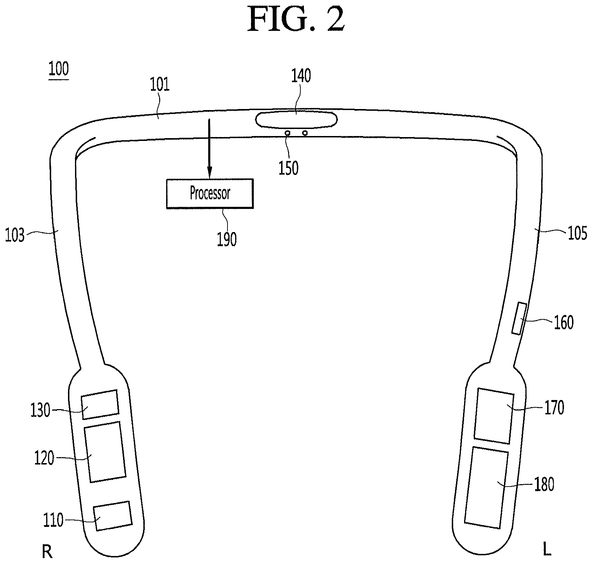

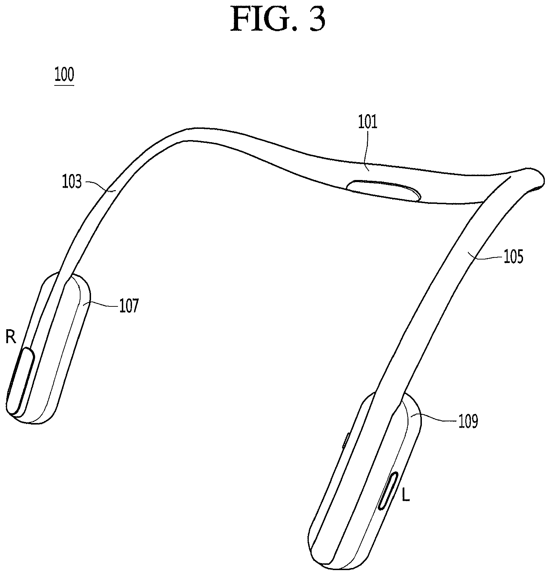

FIG. 2 is a rear view of the emergency response device 100 according to one implementation of the present disclosure. FIG. 3 is a perspective view of an emergency response device 100 according to one implementation of the present disclosure.

Referring to FIG. 2, the emergency response device 100 may include at least one vibration module 110, at least one control button 120, at least one input and output module 130, a battery module 140, a wearing-detection sensor 150, a power button 160, a sensing module 170, a communication module 180, and a processor 190.

The vibration module 110 may output vibration when movement of the user is not detected for a predetermined duration.

The vibration module 110 may output the vibration when an emergency event is detected.

The vibration module 110 may be mounted on a right bent portion 103 as described later.

The control button 120 may be configured for notifying the management server 200 or the mobile device 300 of an emergency event. The control button 120 may be mounted on the right bent portion 103.

When the emergency response device 100 is mounted on the user's neck, the control button 120 may be located on a right breast region of the user. In this case, the user may press the control button 120 quickly with his/her right hand.

Further, the control button 120 is configured to be pressed down by a palm when the emergency response device 100 is mounted on the user's neck, and a garment is placed on the emergency response device 100.

A size of the control button 120 may be 40 mm x 15 mm, that is, may be sized that the control button can be pushed by the palm of the hand. However. this is only an example.

The control button 120 may have an anti-slip pattern to prevent slippage on the garment.

The input and output module 130 may include a speaker for outputting sound, and a microphone for receiving sound.

The input and output module 130 may be mounted on the right bent portion 130. When the user wears the emergency response device 100, the input and output module 130 is located on a user's right clavicle portion and may be located near the user's mouth.

The battery module 140 may supply power to the emergency response device 100. The battery module 140 may be powered by the mounting device on which the emergency response device 100 is mounted.

The battery module 140 may receive power wirelessly from the mounting device via self-resonant or electromagnetic induction.

The battery module 140 may be seated on a center of the middle neck portion 101 for weight distribution.

The wearing-detection sensor 150 may be configured for detecting whether or not the emergency response device 100 is worn on the user. The wearing-detection sensor 150 may be a proximity sensor. However, this is merely an example.

The wearing-detection sensor 150 may be seated on the middle neck portion 101, and may be disposed below the battery module 140.

The power button 160 may be configured for turning power of the emergency response device 100 on or off. When the user pushes the button 160 in a first direction, the power of the emergency response device 100 may be turned on. When the button 160 is pushed in a second direction, the power of the device 100 may be turned off.

The power button 160 may be located on the left bent portion 150.

The sensing module 170 may include a plurality of sensors. The plurality of sensors may include an acceleration sensor, an angular velocity sensor, an air pressure sensor, a heart rate sensor, a magnetic sensor, and a position detection sensor.

The position detection sensor may be configured as a GPS module.

The communication module 180 may communicate with the management server 200 or the mobile device 300. The communication module 180 may transmit information to or receive information from the management server 200 or the mobile device 300 via a short-range wireless communication protocol or the long-range wireless communication protocol.

The processor 190 may control the vibration module 110, the control button 120, the input and output module 130, the battery module 140, the wearing-detection sensor 150, the power button 160, the sensing module 170, and the communication module 180.

That is, the processor 190 may control all operations of the components of the emergency response device 100.

Specific functions of the processor 190 will be described later.

The emergency response device 100 may further include the middle neck portion 101, the right bent portion 103, and the left bent portion 105 for seating the components as described above thereon.

In one implementation, the vibration module 110, the control button 120, and the input and output module 130 may be seated on the right bent portion 103. Specifically, the vibration module 110, the control button 120, and the input and output module 130 may be exposed from a front surface of the right bent portion 103.

The battery module 140 and the wearing-detection sensor 150 may be seated on the middle neck portion 101. The battery module 140 and the wearing-detection sensor 150 may be exposed from a rear surface of the middle neck portion 101.

The power button 160, the sensing module 170, and the communication module 180 may be seated on the left bent portion 105. The power button 160, the sensing module 170, and the communication module 180 may be exposed from a front surface of the left bent portion 105.

Referring to FIG. 3, the middle neck portion 101 may extend rightward and may connect to the right bent portion 103. The middle neck portion 101 may extend leftward and may connect to the left bent portion 105.

The middle neck portion 101 may have a curved cross sectional shape to allow the user to feel a soft touch with the user's neck.

The right bent portion 103 and the left bent portion 105 may extend downwardly of the middle neck portion 101.

The right bent portion 103 and the left bent portion 105 may further have right and left tight-contacting portions 107 and 109 at ends thereof respectively. Each of the tight-contacting portions 107 and 109 may be intended to improve the tight-contacting level of the device 100 with the user's body. Each of the tight-contacting portions 107 and 109 may have a pattern for improving the tight-contacting level of the device 100 with the user's body. Each of the tight-contacting portions 107 and 109 may be made of rubber material.

Next, the mounting device, which the emergency response device 100 may be mounted on, will be described.

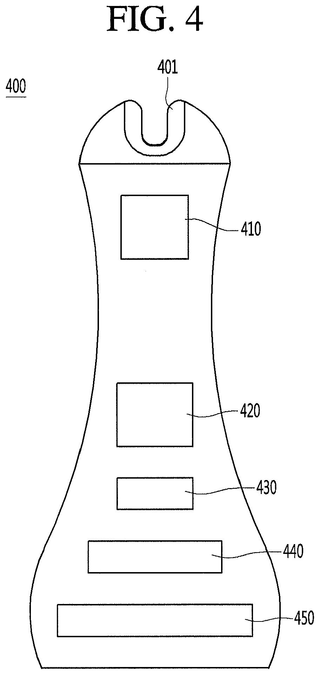

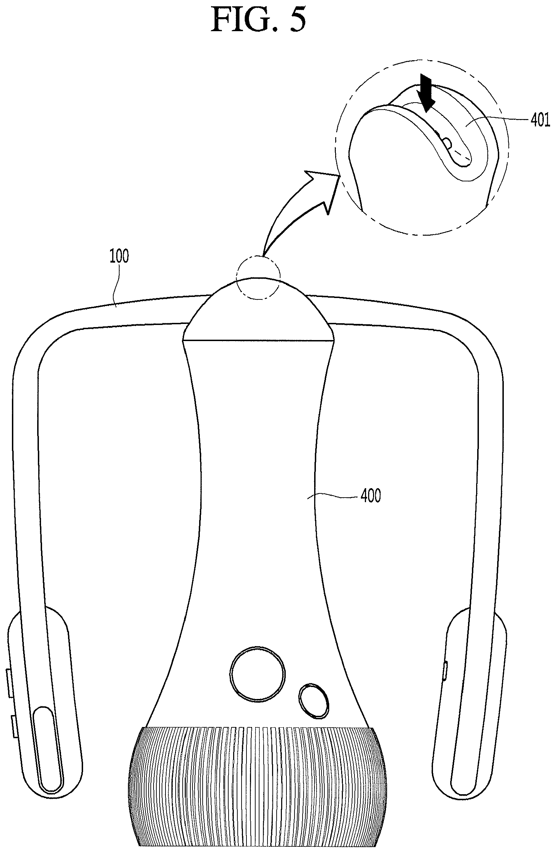

FIG. 4 is a diagram for illustrating a configuration of the mounting device on which the emergency response device is mounted according to one implementation of the present disclosure. FIG. 5 is an illustration of the emergency response device mounted on the mounting device according to one implementation of the present disclosure.

Referring to FIG. 4 and FIG. 5, the mounting device 400 may include a charging module 410, a control button 420, a communication module 430, and input and output module 440, and a battery 450.

The mounting device 400 may have a gourd bottle shape.

The mounting device 400 may have a groove 401 defined in a top thereof such that the emergency response device 100 is received in the groove. The charging module 410 may be disposed below the groove 401.

The charging module 410 may charge the battery module 140 provided in the emergency response device 100. The charging module 410 may be disposed in an upper portion of the mounting device 400. As the emergency response device 100 is mounted into the groove 401, the emergency response device 100 may be easily mounted on the mounting device 400, and may be charged quickly via the charging module 410.

The charging module 410 may supply power to the battery module 140 in a wired manner or wirelessly.

The control button 420 may be configured for controlling the operation of the mounting device 400.

The communication module 430 may be configured for performing wireless communication with the emergency response device 100, the management server 200, or the mobile device 300. The communication module 430 may support a short range communication protocol such as Bluetooth or Wi-Fi.

The input and output module 440 may include a speaker and a microphone. The input and output module 440 may be used to perform a call with a user indoors distant away from the mounting device.

The battery 450 may provide power to the mounting device 400. In order to allow the emergency response device 100 to work even in a power outage situation, the battery 450 may provide power to the emergency response device 100 via the charging module 410.

Referring to FIG. 5, the emergency response device 100 is shown as mounted on the mounting device 400.

The emergency response device 100 and the mounting device 400 may be provided primarily in a day care center for the care of the elderly. However, the present disclosure is not limited thereto. The emergency response device 100 and the mounting device 400 may be provided into various sites such as hospitals, and homes.

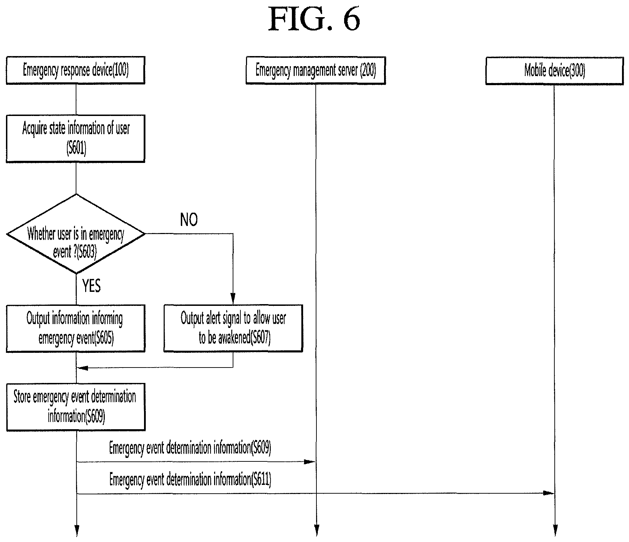

FIG. 6 is a sequence diagram for illustrating a method for operating the emergency response system works according to one implementation of the present disclosure.

The emergency response device 100 acquires state information of the user S601.

In one implementation, the user's state information may include at least one of a heart rate, a respiration rate, a user posture, a user sleep state, a user step count, and an outdoor duration.

The sensing module 170 of the emergency response device 100 may sense the state information of the user. For example, a heart rate sensor may sense a user's heart rate.

The angular velocity sensor may detect the user's waist flexion state.

According to one implementation of the present disclosure, before the emergency response device 100 acquires the user's state information, the device 100 may output monitoring information of the emergency response device 100.

The monitoring information may include an amount of charge of the battery module 140 of the emergency response device 100, and a communication state of the communication module 180 of the device 100.

The emergency response device 100 may output the monitoring information via the speaker of the input and output module 130.

When the emergency response device 100 is disconnected from the emergency management server 200 or mobile device 300 via the communication module 180, the device 100 may output a voice indicating that the communication is disconnected.

The emergency response device 100 may be configured such that when the charge amount of the battery module 140 is lower than a reference charge amount, the device may output a voice indicating that the battery is insufficient.

The emergency response device 100 determines whether the user is in an emergency event based on the acquired state information of the user in operation S603.

When the emergency response device 100 determines that the user is in an emergency event, the device 100 outputs information informing the emergency event at operation S605.

In one implementation, when the user's heart rate exceeds a reference heart rate for a certain period of time, the emergency response device 100 may determine that the user is in the emergency event.

In still another implementation, when the control button 120 is selected, the emergency response device 100 may determine that the user is in an emergency event. In this case, the state information of the user may include information indicating that the control button 120 has been selected for a certain period of time.

In still another implementation, when the movement of the user is not detected for a certain period of time, the emergency response device 100 may determine that the user is in an emergency event. In this connection, the acceleration sensor of the sensing module 170 may sense information on the movement of the emergency response device 100.

In still another implementation, when the movement of the emergency response device 100 is not detected for a certain period of time and the heart rate is below the reference heart rate, the processor 190 of the emergency response device 100 may determine that the user is in an emergency event.

In still another implementation, the emergency response device 100 detects falling down or collapsing of the user, the emergency response device 100 may check the user's state of consciousness via the user's heart rate.

When the user's state is an unconscious state, the emergency response device 100 may detect that this is an emergency event.

In one implementation, when the emergency response device 100 determines that the user is in an emergency event, the emergency response device 100 may automatically connect a call to a guardian mobile device 300. The emergency response device 100 may automatically store a telephone number of the mobile device 300 to be connected thereto. To this end, the emergency response device 100 may further include a memory (not shown).

A plurality of telephone numbers may be stored. In this case, when no telephone connection is made to a mobile device 300 corresponding to a first-ranked phone number, the emergency response device 100 may automatically perform a telephone communication to a mobile device 300 corresponding to a second-ranked telephone number.

In still another implementation, when the emergency response device 100 determines that the user is in an emergency event, the emergency response device 100 may output a voice having a predetermined intensity or greater. The emergency management server 200 may determine a location of the emergency response device 100 outputting the voice. Thus, the location of the user may be confirmed to quickly respond to an emergency event of a user.

In still another implementation, when the user is in an emergency event occurring at a high floor of the building, the emergency response device 100 may check an approximate location of the user via the GPS module, and then the emergency response device 100 may be able to figure out which floor in the building the user is located correctly via an air pressure sensor.

There may be pressure differences between floors in the building. Thus, the emergency response device 100 may determine what floor in which the user is located based on an atmospheric pressure measurement obtained via an atmospheric pressure sensor at the approximate location of the identified user.

The emergency response device 100 may transmit the final location of the user as obtained to one or more of the emergency management server 200 or the mobile device 300. A manager for managing the emergency management server 200, or the guardian having the mobile device 300 may quickly search for the user of the emergency response device 100 via the final location of the emergency response device 100.

In still another implementation, the emergency response device 100 may learn and store information about a location at which the user usually lies. When the user's current lying location differs from the stored lying location, an abnormality signal may be generated to indicate a signal requesting a rescue.

The emergency response device 100 may send the abnormality signal to the emergency management server 200 or the mobile device 300.

In still another implementation, when the emergency response device 100 is located a certain distance away from the guardian mobile device 300, the emergency response device 100 may send a message to the guardian's mobile device 300 to inform this situation. In response to reception of the message, the mobile device 300 may output a notification indicating that the user of the emergency response device 100 has departed from the predefined location.

When the user is determined not to be in an emergency event, the emergency response device 100 outputs a alert signal to allow the user to be awakened S607.

In one implementation, when the movement of the user is not detected for a predetermined time, the emergency response device 100 may output the vibration via the vibration module 110 or may output the sound via the input/output module 130. The movement of the user may correspond to the movement of the emergency response device 100. Further, the predetermined time may be one hour, but this is only an example.

Thereafter, when the user's heart rate is within the reference heart rate range, the emergency response device 100 may determine that the user is not in an emergency event.

In one example, when the user's heart rate is not within the reference heart rate range after outputting the information to allow the user to be awakened, the emergency response device 100 may output information informing an emergency event as in operation S605.

In still another implementation, when the user does not react within a certain period of time after outputting the information to allow the user to be awakened via the vibration, the emergency response device 100 may output information informing an emergency event. The output information may be transmitted to the emergency management server 200 or the mobile device 300. In this connection, the term "reaction" from the user may indicate inputting a voice via the microphone of the input and output module 130 or selecting the control button 120.

The emergency response device 100 stores emergency event determination information used for determination of an emergency event S609. The device transmits the emergency event determination information to the emergency management server 200 or the mobile device 300 at S611.

The present disclosure as described above may be implemented using a computer readable code on a computer readable medium on which a computer readable program is recorded. The computer readable medium includes all kinds of storing devices in which data that may be read by a computer system is stored. Examples of the medium that can be read by a computer include HDD (Hard Disk Drive), SSD (Solid State Disk), SDD (Silicon Disk Drive), ROM, RAM, CD-ROM, magnetic tape, floppy disk, optical data storage, etc. The medium may be, for example, implemented in a form of a carrier wave (for example, for transmission over the Internet). Further, the computer may include the controller 180 of the present device 100. Accordingly, the above description should not be construed in a limiting sense in all respects and should be considered illustrative. The scope of the present disclosure shall be determined by rational interpretation of the appended claims. All changes within the equivalent range of the present disclosure are included in the scope of the present disclosure.

## Claims

1. An emergency response device wearable on a user's neck, the device comprising:
a communication module configured to communicate with an emergency management server or a mobile device;
a sensing module configured to sense state information of a user while the user is wearing the emergency response device; and
a processor configured to:
determine, based on the sensed state information of the user, whether the user is in an emergency event; and
upon determination that the user is in an emergency event, output information indicating the emergency event.

2. The emergency response device of claim 1, wherein the processor is further configured to:
upon determination that the user is not in an emergency event, output an alert signal to allow the user to be awakened; and
upon non-reception of a reaction to the alert signal from the user within a predetermined time after outputting the alert signal, output a vibration indicating the emergency event.

3. The emergency response device of claim 2, wherein the device further comprises a vibration module, wherein the processor is further configured to output the alert signal using the vibration module.

4. The emergency response device of claim 1, wherein the processor is further configured to output the information indicating the emergency event to the emergency management server or the mobile device.

5. The emergency response device of claim 1, wherein the device includes:
a middle neck portion having a curved cross sectional shape;
a right bent portion bent downwardly from a right end of the middle neck portion; and
a left bent portion bent downwardly from a left end of the middle neck portion.

6. The emergency response device of claim 5, wherein the device includes:
an input and output module including a speakers and a microphone; and
a battery module mounted on the middle neck portion for supplying power to the emergency response device.

7. The emergency response device of claim 6, wherein the input and output module is mounted on the right bent portion, wherein the battery module is mounted in a center region of the middle neck portion.

8. An emergency response device wearable on a user's neck, the device comprising:
a communication module configured to communicate with an emergency management server or a mobile device;
a sensing module configured to sense state information of a user while the user is wearing the emergency response device; and
a processor configured to output information indicating an emergency event when a movement of the user is not sensed for a predetermined time duration and when the user's heart rate is lower than a reference heart rate.

9. The emergency response device of claim 8, wherein the processor is further configured to:
upon determination that the user is not in an emergency event, output an alert signal to allow the user to be awakened; and
upon non-reception of a reaction to the alert signal from the user within a predetermined time after outputting the alert signal, output a vibration indicating the emergency event.

10. The emergency response device of claim 9, wherein the device further comprises a vibration module, wherein the processor is further configured to output the alert signal using the vibration module.

11. The emergency response device of claim 8, wherein the processor is further configured to output the information indicating the emergency event to the emergency management server or the mobile device.

12. The emergency response device of claim 8, wherein the device includes:
a middle neck portion having a curved cross sectional shape;
a right bent portion bent downwardly from a right end of the middle neck portion; and
a left bent portion bent downwardly from a left end of the middle neck portion.

13. The emergency response device of claim 12, wherein the device includes:
an input and output module including a speakers and a microphone; and
a battery module mounted on the middle neck portion for supplying power to the emergency response device.

14. The emergency response device of claim 13, wherein the input and output module is mounted on the right bent portion, wherein the battery module is mounted in a center region of the middle neck portion.
